# EUROPÄISCHE PATENTSCHRIFT

(11) **EP 3 562 522 B1**
(45) Veröffentlichungstag und Bekanntmachung des Hinweises auf die Patenterteilung: **13.01.2021**
(21) Anmeldenummer: 17822663.5
(22) Anmeldetag: 20.12.2017
(51) Int. Cl.: A61L 27/04, A61L 27/54, A61L 27/58, A61L 31/02, A61L 31/14, A61L 31/16

(54) **ANORDNUNG MIT EINEM RESORBIERBAREN MATERIAL MIT ANTIBAKTERIELLER WIRKUNG**
ASSEMBLY COMPRISING A RESORBABLE MATERIAL HAVING ANTIBACTERIAL ACTIVITY
DISPOSITIF COMPORTANT UN MATÉRIAU RÉSORBABLE À ACTION ANTIBACTÉRIENNE

(30) Priorität: 28.12.2016 DE 102016125816; 20.03.2017 DE 102017204627
(43) Veröffentlichungstag der Anmeldung: 06.11.2019
(73) Patentinhaber: Meotec GmbH, 52068 Aachen (DE)
(72) Erfinder: KOPP, Alexander, 52064 Aachen (DE); D'ELIA, Francesco, 52064 Aachen (DE); SMEETS, Ralf, 20249 Hamburg (DE)
(74) Vertreter: FARAGO Patentanwälte
(86) Internationale Anmeldenummer: PCT/EP2017/083826
(87) Internationale Veröffentlichungsnummer: WO 2018/122066

(56) Entgegenhaltungen:
- WO-A1-2013/176769
- US-A- 5 765 740
- DATABASE WPI Week 201542 Thomson Scientific, London, GB; AN 2015-36402J XP002778840, & CN 104 511 048 A (UNIV SHANGHAI JIAO TONG NINTH PEOPLES HO) 15. April 2015 (2015-04-15) -& DATABASE EPODOC [Online] EUROPEAN PATENT OFFICE, THE HAGUE, NL; XP002778841, Database accession no. CN-201310451040-A -& CN 104 511 048 A (JIAOTONG 9TH PEOPLES HOSPITAL) 15. April 2015 (2015-04-15)
- DATABASE WPI Week 201544 Thomson Scientific, London, GB; AN 2015-363036 XP002778861, & CN 104 513 922 A (UNIV SHANGHAI JIAO TONG NINTH PEOPLES HO) 15. April 2015 (2015-04-15) -& DATABASE EPODOC [Online] EUROPEAN PATENT OFFICE, THE HAGUE, NL; XP002778862, Database accession no. CN-201310454404-A -& CN 104 513 922 A (JIAOTONG 9TH PEOPLES HOSPITAL) 15. April 2015 (2015-04-15)
- DATABASE WPI Week 201636 Thomson Scientific, London, GB; AN 2016-13575H XP002778884, & CN 105 349 858 A (UNIV CENT SOUTH) 24. Februar 2016 (2016-02-24) -& DATABASE EPODOC [Online] EUROPEAN PATENT OFFICE, THE HAGUE, NL; XP002778885, Database accession no. CN-201510833664-A -& CN 105 349 858 A (UNIV CENTRAL SOUTH) 24. Februar 2016 (2016-02-24)
- LI YANG ET AL: "Biodegradable Mg-Cu alloy implants with antibacterial activity for the treatment of osteomyelitis:In vitroandin vivoevaluations", BIOMATERIALS, ELSEVIER SCIENCE PUBLISHERS BV., BARKING, GB, Bd. 106, 20. August 2016 (2016-08-20), Seiten 250-263, XP029718652, ISSN: 0142-9612, DOI: 10.1016/J.BIOMATERIALS.2016.08.031
- MUHAMMAD IMRAN RAHIM ET AL: "Alkalization is responsible for antibacterial effects of corroding magnesium : ANTIBACTERIAL ACTIVITY OF MAGNESIUM", JOURNAL OF BIOMEDICAL MATERIALS RESEARCH. PART A, Bd. 103, Nr. 11, 24. Mai 2015 (2015-05-24) , Seiten 3526-3532, XP055456985, HOBOKEN, NY, US ISSN: 1549-3296, DOI: 10.1002/jbm.a.35503
- N.N.: "Magnesium Goodfellow Catalogue", Goodfellow , XP002778870, Gefunden im Internet: URL:http://www.goodfellow.com/pdf/4611_111 1010.pdf [gefunden am 2018-03-07]
- DATABASE WPI Week 201429 Thomson Scientific, London, GB; AN 2014-H04779 XP002778886, & CN 103 614 601 A (SUZHOU ORIGIN MEDICAL TECHNOLOGY CO LTD) 5. März 2014 (2014-03-05) -& DATABASE EPODOC [Online] EUROPEAN PATENT OFFICE, THE HAGUE, NL; XP002778887, Database accession no. CN-201310687355-A
- FRANK WITTE: "The history of biodegradable magnesium implants: A review☆", ACTA BIOMATERIALIA, Bd. 6, Nr. 5, 1. Mai 2010 (2010-05-01), Seiten 1680-1692, XP055163222, ISSN: 1742-7061, DOI: 10.1016/j.actbio.2010.02.028
- DATABASE WPI Week 201707 Thomson Scientific, London, GB; AN 2016-809156 XP002778909, & KR 2016 0142667 A (REXWELL CO LTD) 13. Dezember 2016 (2016-12-13) -& DATABASE EPODOC [Online] EUROPEAN PATENT OFFICE, THE HAGUE, NL; XP002778910, Database accession no. KR-20150078702-A
- DATABASE WPI Week 201213 Thomson Scientific, London, GB; AN 2012-B96197 XP002778913, & CN 202 122 594 U (FOOSIN MEDICAL SUPPLIES LTD) 25. Januar 2012 (2012-01-25) -& DATABASE EPODOC [Online] EUROPEAN PATENT OFFICE, THE HAGUE, NL; XP002778914, Database accession no. CN-201120191625-U

## Beschreibung

### Gebiet der Erfindung

Die Erfindung bezieht sich auf eine Anordnung umfassend mindestens ein Bauteil aus einem resorbierbaren Material mit antibakterieller Wirkung und auf eine Halterungsform zur Bereitstellung dieser Anordnung.

### Hintergrund der Erfindung

Aufgrund von Kontamination, beispielsweise durch das kurzweilige oder langfristige Einbringen eines Fremdkörpers, kann es im Körper eines Lebewesens zu Entzündungsreaktionen kommen. Insbesondere bei solchen Medizinprodukten, welche mit dem ansonsten sterilen Knochen in Kontakt kommen, entstehen häufig solche Entzündungen, welche bekämpft werden müssen oder andernfalls das Einwachsverhalten von Implantaten verschlechtern bzw. manchmal auch den Organismus als Ganzes gefährden. Eine Knochenentzündung ist eine Entzündung der Knochensubstanz. Im Alltagsgebrauch wird der Begriff Knochenentzündung verallgemeinernd für die Entzündung der äußeren, harten Knochensubstanz sowie des Knochenmarks verwendet. Mediziner unterscheiden jedoch genauer zwischen einer Entzündung der harten Substanz des Knochens (Osteitis) ohne Beteiligung des Knochenmarks und einer Entzündung des Knochenmarks (Osteomyelitis). Osteitis und Osteomyelitis werden in den meisten Fällen durch Bakterien (ganz selten durch Viren oder Pilze) verursacht und treten nach Knochenbrüchen (Frakturen), Operationen an Knochen oder Infektionen auf. Zu einer Knochenentzündung kommt es, wenn Bakterien von außen den Knochen erreichen, beispielsweise bei einer offenen Verletzung oder einer OP-Wunde. Welche Knochen genau betroffen sind, richtet sich nach dem Ort der ursächlichen Verletzung. Betroffen sind häufig Oberschenkel (Femur) und Unterschenkel (Tibia).

Ein häufig auftretender Sonderfall der vorbeschriebenen Entzündungen ist die Periimplantitis. Diese bezeichnet eine Entzündung der Weich- und Hartgewebe des Implantatbetts um dentale Implantate herum. Das Vorstadium der Periimplantitis ist die Mukositis, eine Entzündung der den Implantathals umgebenden Schleimhaut. Periimplantitis macht sich durch Blutungen und fortschreitenden Knochenverlust um das Implantat herum bemerkbar. Falls unbehandelt führt dies zu einer fortschreitenden Beweglichkeit des Implantats und zu einem möglichen Implatatversagen mangels ausreichender noch vorhandener Befestigung des Implantats. Periimplantitische Entzündungen werden bei 10% - 45% aller Dentalimplantate innerhalb von 10 Jahren nach deren Implantation festgestellt. Somit stellt die Periimplantitis ein anhaltendes medizinisches Problem dar, das nicht einfach zu behandeln ist. Die Entstehung und Fortschreitung der Periimplantitis ist dabei auf das Vorhandensein von bakteriellen Biofilmen zurückzuführen. Das Wachsen von Biofilmen stellt die bevorzugte Vermehrungsmethode für die meisten Bakterien dar und trägt zu deren Resistenzbildung bei.

Es hat sich gezeigt, dass Silber- und Silberlegierungen effektiv zur Unterdrückung der Bakterienvermehrung eingesetzt werden können, da diese Materialien die DNA von sowohl grampositiven als auch gramnegativen Bakterien zerstören. Zur Hinzugabe von Silber in unterschiedlichen Zuständen zu Titanimplantaten wurden diverse Techniken wie beispielsweise Ionen-Implantation, physikalischen Gasphasenabscheidungen (PVD), Sputtern und plasma-elektrolytische Oxidation getestet und die Eignung von Silber als antibakterielle Beschichtung zur Verhinderung von Knochenverlust aufgrund von Periimplantitis festgestellt. Aber auch wenn die Verwendung von Silberbeschichtungen die Anhaftung und Kolonisation von Bakterien verhindert, so erhöhen solche Beschichtungen die Oberflächenrauigkeit von Implantaten. Eine erhöhte Rauigkeit beispielsweise von mehr als 0.2µm ist dafür bekannt, dass es die Bildung von Biofilmen unterstützt und daher zu einem spontanen Fortschreiten der Periimplantitis beitragen kann.

Magnesium-Silber-Legierungen wurden ebenfalls als antibakteriell wirkende Implantate untersucht. Diese Legierung war korrosionsbeständig und zeigte zufriedenstellende mechanischen Eigenschaften sowie eine vernachlässigbare Zytotoxizität und eine antibakterielle Aktivität. Resorbierbare Materialien scheiden allerdings für manche Implantate aus, da diese nicht langzeitig stabil sind und beispielweise dentale Implantate als permanente Einsätze vorgesehen sind.

Es wäre daher wünschenswert, ein geeignetes Mittel zur Verfügung zu haben, mit dem Implantate einfach und ohne aufwendige Modifikation bereitgestellt werden können, und bei denen nach Implantation dennoch Gefahr einer Entzündung, beispielsweise die Periimplantitis, wirksam unterdrückt oder bekämpft werden kann.

CN105349858A [DATABASE WPI, Week 201636; AN 2016-13575H / XP002778884] beschreibt Implantate aus (bio)degradierbaren, antibakteriellen Magnesiumlegierungen mit 0,5-4 Gew.-% Silber, sowie 0,5-4 Gew.-% Yttrium.

CN103614601A [DATABASE WPI, Week 201429; AN 2014-H04779 / XP002778886] beschreibt Magnesiumlegierungen für Implantate, enthaltend 0,05 bis 8,0 Gew. % Silber, 0,005 bis 6,5 Gew.% Zink und 0,1 bis 2,0 Gew.% Mangan.

### Zusammenfassung der Erfindung

Es ist daher eine Aufgabe der vorliegenden Erfindung, ein geeignetes Mittel zur Verfügung zu stellen, welches einfach und ohne aufwendige Modifikation bereitgestellt werden kann und das dazu geeignet ist, nach dem Einbringen eine Entzündungsreaktion im Körper eines Lebewesens wirksam zu unterdrücken oder zu bekämpfen.

Die Erfindung wird durch die Ansprüche definiert.

Diese Aufgabe wird gelöst durch eine Anordnung zur Verwendung als Gewebeexpander, Hautexpander oder Implantat mit antibakterieller Wirkung umfassend mindestens ein Bauteil aus einem resorbierbaren Material mit antibakterieller Wirkung mit einer Form, die ein Aspektverhältnis größer 10 besitzt und wobei das Material eine schnell korrodierende Magnesiumlegierung ist, die a. einen Silberanteil von zwischen 6% und 12%, und b. einen Eisenanteil von zwischen 0,01% und 1% oder einen Kupferanteil von zwischen 0,1 und 10% umfasst.

Das Aspektverhältnis bezeichnet hierbei das Verhältnis aus der Höhe einer Struktur zu ihrer kleinsten lateralen Ausdehnung. Bei einer fadenförmigen Struktur entspricht die Höhe der Struktur die Fadenlänge und die kleinste laterale Ausdehnung dem Durchmesser des Querschnitts der fadenförmigen Struktur senkrecht zur Länge. Ein Bauteil mit Aspektverhältnis mindestens 10 bezeichnet hier ein fadenförmiges Bauteil, das beispielsweise durch Drahtziehen, Extrusion, Stanzen oder Laser- oder Wasserstrahlschneiden hergestellt werden kann.

Ein Material wird als resorbierbar bezeichnet, wenn sich das Material in einer Körperumgebung im jeweiligen Organismus auflöst und das Material vom Organismus zumindest zum Teil aufgenommen wird. Anordnungen aus Magnesium sind somit hervorragend zum Einsatz als Implantat geeignet. Hierbei ist die Resorption sowohl eine materialspezifische Eigenschaft als auch eine Funktion der Zeit. Magnesium ist sehr gut im menschlichen Körper resorbierbar, da es als Mineral in fast allen Körperzellen vorhanden ist und Magnesium oder Legierungen aus Magnesium deutlich fester sind als beispielsweise resorbierbare Polymere. Die erfindungsgemäße Anordnung ist zur Deposition im menschlichen Körper vorgesehen und stellt daher ein unterstützendes Implantat dar. Solche Magnesium-Implantate können somit vom Körper absorbiert werden. Somit verbleiben Implantate aus Magnesium nicht mehr als Fremdkörper im menschlichen Körper und es werden so eventuelle spätere Komplikationen vermieden.

Unter einer Magnesiumlegierung wird ein Material verstanden, welches überwiegend aus Magnesium besteht, aber auch andere Materialien enthalten kann. Diese können sowohl einen geringen Anteil aufweisen, beispielsweise kleiner 0,1 Gewichtsprozent, als auch in höherem Anteil größer 0,1 Gewichtsprozent enthalten sein. Auch reines Magnesium ohne andere Bestandteile, oder nur aufweisend einen verschwindend geringen Anteil solcher, wird in diesem Sinne als Magnesiumlegierung verstanden (nicht erfindungsgemäß).

Der Begriff "Korrosion" bezeichnet eine Reaktion eines Werkstoffs mit seiner Umgebung, die eine Veränderung des Werkstoffs bewirkt und den Werkstoff zersetzt oder zerfrisst. Eine durch Lebewesen verursachte Korrosion wird als Biokorrosion bezeichnet. Die Korrosionsrate ist die Geschwindigkeit der Materialveränderung bzw. des Materialabtrags und bestimmt die Schnelligkeit der Korrosion. Sie hängt wesentlich von der Konzentration der beteiligten Stoffe, dem pH-Wert der Umgebung und der Temperatur ab. Hier bezieht sich der Ausdruck schnell korrodierend auf die Materialveränderung in wässriger Umgebung bei pH-Werten zwischen 4 und 8 (sauer bis neutral bzw. leicht alkalisch) bei Raumtemperatur bis Körpertemperatur (37 - 38 °C). Als schnell wird eine Korrosion im Sinne der vorliegenden Erfindung verstanden, wenn die normierte oder flächen- oder volumenabhängige beschleunigte oder reale in-vitro oder invivo Korrosionsrate mehr als 1 mm Verlust an Materialdicke pro Jahr (in mm/Jahr) beträgt. In diesem Sinne werden auch höhere Korrosionsraten, beispielsweise mehr als 2 mm/Jahr, 3 mm/Jahr, 4 mm/Jahr, 5 mm/Jahr oder 10 mm/Jahr als schnell korrodierend verstanden. Gewöhnlich wird im Stand der Technik beispielsweise der Eisengehalt in Magnesiumlegierungen auf ein Minimum reduziert, beispielsweise kleiner 50ppm, um eine Korrosion der betreffenden Magnesiumlegierung zu verhindern.

Ein Material oder eine Substanz mit antibakterieller Wirkung reduziert die Vermehrungswirkung von Mikroorganismen (Bakterien), tötet sie ab oder inaktiviert sie, beispielsweise durch biologische, chemische, mechanische oder thermische Schädigung.

Beim Abbau von Magnesium in einem wässrigen Milieu wie dem Körper entsteht reaktionsbedingt immer Wasserstoffgas. In geringen Mengen, beziehungsweise bei moderater Freisetzung, hat dies jedoch keine Effekte. Wenn der Abbau hinreichend schnell und / oder durch die Größe des Bauteils auf einer hinreichend großen Fläche abläuft und eine gewisse Menge an Wasserstoffgas, beziehungsweise eine ausreichend hohe Freisetzungsrate erreicht wird, wirkt bereits die Menge an Wasserstoffgas antibakteriell. Durch die schnelle Korrosion der Magnesiumlegierung wird die für die antibakterielle Wirkung benötigte Menge an Wasserstoffgas erzeugt. Findet der Abbau zu exzessiv und darüber hinaus in verschlossenen Gewebeabschnitten statt, kann die Freisetzung darüber hinaus zur Bildung von Gastaschen führen, welche zumeist nicht erwünscht und somit vermieden werden müssen. Deshalb muss der Abbau des Implantates beziehungsweise die Korrosionsrate genau auf die Funktion abgestimmt werden. Bei einer langsamen Korrosion wäre die antibakterielle Wirkung der Magnesiumlegierung nicht ausreichend, um Entzündungen wie beispielsweise die Periimplantitis zu vermindern oder gar zu unterdrücken.

Die erfindungsgemäße Anordnung stellt somit ein geeignetes Mittel dar, das als eigenes Implantat oder zusammen mit anderen Implantaten einfach und ohne aufwendige Modifikation der anderen Implantate bereitgestellt werden kann, und das dazu geeignet ist, keine Entzündungen wie beispielsweise Periimplantitis selber zu verursachen und nach Implantation des anderen Implantats unter anderem die Entzündungsreaktion, die sonst durch das andere Implantat möglicherweise verursacht wird, wirksam zu unterdrücken oder zu bekämpfen. Die erfindungsgemäße Anordnung kann auch prophylaktisch zur Entzündungsvorbeugung bei dentalen Implantaten oder bei Implantaten an anderen Körperpositionen verwendet werden.

Beispielsweise kann die erfindungsgemäße Anordnung direkt angrenzend oder in der Nähe eines entzündeten Gewebes, oder eines potenziell entzündungsgefährdeten Gewebes, beispielsweise Knochen, eingebracht werden, indem es bereits eine bevorzugte Form aufweist oder einfach in mehreren Lagen oder durch wirres Abwickeln und Anordnen eingebracht wird.

Erfindungsgemäß umfasst die Magnesiumlegierung einen Silberanteil von zwischen 6% und 12%, vorzugsweise zwischen 6% und 10%, wobei sich die Prozentangabe auf Gewichtsprozente bezieht. Auch Silber besitzt solch eine antibakterielle Wirkung. Durch die schnelle Korrosion der Magnesiumlegierung gemäß der vorliegenden Erfindung werden Silber-Ionen in ausreichender Menge aus der Magnesiumlegierung freigesetzt, die dann von der umgebenden Schleimhaut resorbiert werden und dort wie voranstehend beschrieben ihre antibakterielle Wirkung im Lebewesen freisetzen. Bei Freisetzung von Silber-Ionen wirkt sich der antibakterielle Schutz um den Ort der erfindungsgemäßen Anordnung herum im Körper des Lebewesens, in dem sich die Anordnung befindet, aus. Um einen guten Schutz gegen Infektionen, insbesondere die Periimplantitis oder die Perimucositis, aber auch andere Entzündungen bieten zu können, sollte die Anordnung in der Nähe des Entzündungsherdes, beispielsweise bei dentalen Anwendungen nahe am Zahnfleischrand und damit nahe am Ort möglicher Infektionen platziert werden. Über den Silberanteil lässt sich die gewünschte antibakterielle Wirkung sehr genau dosieren.

Erfindungsgemäß umfasst die Magnesiumlegierung einen Eisenanteil von zwischen 100ppm und 1%, oder einen Kupferanteil von zwischen 0,1 und 10%. Üblicherweise wird bei resorbierbaren Magnesiumlegierungen versucht, den Anteil an Verunreinigungen wie Eisen oder anderen so gering wie möglich zu halten, um einen langsamen Abbau und damit eine lang anhaltende Stützwirkung der zumeist zur Stabilisierung von Knochen genutzten Implantate zu bewirken. Überraschender Weise hat sich aber gezeigt, dass eine schnellkorrodierende Magnesiumlegierung dann von Vorteil ist, wenn statt der Stützwirkung ein antibakterielles Wirken der Abbauprodukte als Zweck des Implantats dienen soll. Eine Magnesiumlegierung mit einer solchen eben hohen Eisenkonzentration stellt eine schnell korrodierende Magnesiumlegierung im Sinne der vorliegenden Erfindung dar und ist daher gut zur Freisetzung von

Wasserstoffgas als auch der Silber-Ionen geeignet. In einer weiteren Ausführungsform ist der Eisenanteil größer 150ppm, in einer anderen Ausführungsform größer 200ppm. In einer besonders bevorzugten Ausführungsform ist der Eisenanteil größer 250ppm. Über den Eisenanteil kann die pro Zeiteinheit mittels Korrosion freigesetzte Menge an Wasserstoffgas beziehungsweise an Silber-Ionen gesteuert und an die notwendige Menge zur Bekämpfung der entsprechenden Entzündung angepasst werden. Eisen als Zusatz zur Korrosionsteuerung ist vorteilhaft gegenüber anderen Zusätzen mit ähnlichen Effekten, da Eisen vom Lebewesen natürlich resorbiert werden kann und somit keine Schäden durch seine Präsenz im Lebewesen verursacht.

Erfindungsgemäß ist der Eisenanteil kleiner 1%. Damit bleibt die Magnesiumlegierung in einer Körperumgebung weiterhin lösbar und stellt somit weiterhin ein resorbierbares Material dar. In einer bevorzugten Ausführungsform ist der Eisenanteil kleiner 0,5%, in einer besonders bevorzugten Ausführungsform ist der Eisenanteil kleiner als 0,1%. Die voranstehenden Prozentangaben beziehen sich auf Gewichtsprozente. Die Löslichkeit von Eisen in Magnesium steigt dabei mit der Temperatur, bei der die Legierung hergestellt wurde.

Erfindungsgemäß umfasst die Magnesiumlegierung einen Kupferanteil von zwischen 0,1 und 10% oder einen Eisenanteil von zwischen 0,01 und 1%. Üblicherweise wird bei resorbierbaren Magnesiumlegierungen versucht, den Anteil an Verunreinigungen wie Kupfer oder anderen so gering wie möglich zu halten, um einen langsamen Abbau und damit eine lang anhaltende Stützwirkung der zumeist zur Stabilisierung von Knochen genutzten Implantate zu bewirken. Überraschender Weise hat sich aber gezeigt, dass eine schnellkorrodierende Magnesiumlegierung dann von Vorteil ist, wenn statt der Stützwirkung ein antibakterielles Wirken der Abbauprodukte als Zweck des Implantats dienen soll. Eine Magnesiumlegierung mit einer solchen eben nicht geringen Kupferkonzentration stellt eine schneller korrodierende Magnesiumlegierung im Sinne der vorliegenden Erfindung im Vergleich zu einer Magnesiumlegierung ohne Kupferanteil dar und ist daher zur Freisetzung von Wasserstoffgas als auch gegebenenfalls zusätzlich vorhandenen Silber-Ionen grundsätzlich geeignet. Ab einer Kupferkonzentration oberhalb von 0,1% ist ein Einfluss auf die Korrosionsrate feststellbar. In einer weiteren Ausführungsform ist der Kupferanteil größer 0,3%. Ab dieser Kupferkonzentration erhält man deutlich schneller korrodierende Magnesiumlegierungen, sodass die vorteilhaften Korrosionseigenschaften ein effektives antibakterielles Wirken der Abbauprodukte sicherstellen. Eine Magnesiumlegierung mit einer solchen hohen Kupferkonzentration stellt eine besonders schnell korrodierende Magnesiumlegierung im Sinne der vorliegenden Erfindung im Vergleich zu einer Magnesiumlegierung ohne Kupferanteil dar und ist daher insbesondere zur Freisetzung von Wasserstoffgas als auch gegebenenfalls zusätzlich vorhandenen Silber-Ionen besonders gut geeignet. In einer anderen Ausführungsform ist der Kupferanteil größer 0,6%. In einer besonders bevorzugten Ausführungsform ist der Kupferanteil größer 1,0%. Die voranstehenden Prozentangaben bezeichnen hier Gewichtsprozente. Über den Kupferanteil kann die pro Zeiteinheit mittels Korrosion freigesetzte Menge an Wasserstoffgas beziehungsweise an Silber-Ionen gesteuert und an die notwendige Menge zur Bekämpfung der entsprechenden Entzündung angepasst werden. Kupfer als Zusatz zur Korrosionsteuerung ist vorteilhaft gegenüber anderen Zusätzen mit ähnlichen Effekten, da Kupfer vom Lebewesen natürlich resorbiert werden kann und somit keine Schäden durch seine Präsenz im Lebewesen verursacht.

Erfindungsgemäß ist der Kupferanteil kleiner 10%. Damit bleibt die Magnesiumlegierung in einer Körperumgebung weiterhin lösbar und stellt somit weiterhin ein resorbierbares Material dar. In einer bevorzugten Ausführungsform ist der Kupferanteil kleiner 5%, in einer besonders bevorzugten Ausführungsform ist der Kupferanteil kleiner als 2%. Die voranstehenden Prozentangaben beziehen sich auf Gewichtsprozente. Die Löslichkeit von Kupfer in Magnesium steigt dabei mit der Temperatur, bei der die Legierung hergestellt wurde.

Die voranstehenden Legierungen mit Kupfer können auch Eisenanteile aufweisen. Beispielsweise können obige Magnesiumlegierungen mit den voranstehenden spezifizierten Kupferanteil einen Eisenanteil größer 100ppm, bevorzugt größer 150ppm, besonders bevorzugt größer 200ppm, noch mehr bevorzugt größer 250ppm, aufweisen. Hierbei ist der Eisenanteil kleiner 1%, bevorzugt kleiner 0,5%, besonders bevorzugt kleiner 0,1 %.

In einer weiteren Ausführungsform umfasst die Magnesiumlegierung als Zusatz ein oder mehrere Elemente der Gruppe Al, Zn, Si, Mn, Ca, Li, Sn, Sr und/oder P mit einem jeweiligen Anteil kleiner 1%, bevorzugt kleiner 0,6%, besonders bevorzugt kleiner 0,2% und einem Gesamtanteil kleiner 2%, bevorzugt kleiner 1,5%, besonders bevorzugt kleiner 0,4%. Die voranstehenden Prozentangaben beziehen sich auf Gewichtsprozente. Die voranstehenden Zusätze sind kompatibel für biomedizinische Anwendungen. Die Hinzugabe der Zusätze in den spezifizierten Mengen erlaubt es, die Magnesiumlegierung mit einer verbesserten Robustheit und größeren Duktilität bereitzustellen, ohne dabei die antibakterielle Wirkung der Magnesiumlegierung zu schwächen. Beispielsweise verbessert die Zugabe von Lithium die Duktilität von Magnesium in einer entsprechenden Legierung. Die Zugabe beispielsweise von Aluminium sollte nicht zu groß sein, da exzessive Aluminiumkonzentrationen mit Alzheimer in Verbindung gebracht werden.

In einer weiteren Ausführungsform umfasst die Magnesiumlegierung als Zusatz ein oder mehrere Elemente der Gruppe Sc, Y, La, Ce, Pr, Nd, Pm, Sm, Eu, Gd, Tb, Dy, Ho, Er, Tm, Yb, und/oder Lu mit einem jeweiligen Anteil kleiner 4,5%, bevorzugt kleiner 2%, besonders bevorzugt kleiner 0,15% umfasst, wobei der Gesamtanteil kleiner 7,5%, bevorzugt kleiner 5%, besonders bevorzugt kleiner 0,4% ist. Die voranstehenden Prozentangaben beziehen sich auf Gewichtsprozente. Seltene Erden haben grundsätzlich ein hohes Lösungsvermögen in einer Magnesiumlegierung und verbessern die mechanischen Eigenschaften der Magnesiumlegierung wie beispielsweise deren Robustheit und mechanische Belastbarkeit, was insbesondere für Implantate wichtig ist.

In einer weiteren Ausführungsform umfasst die Magnesiumlegierung Zirkon mit einem Anteil kleiner 0,5%. Die voranstehenden Prozentangaben beziehen sich auf Gewichtsprozente. Zirkon verkleinert die Korngrößen in Magnesiumlegierungen, insbesondere in nicht-AL-haltigen Magnesiumlegierungen. Der Zirkon-Anteil ist nach oben begrenzt, da eine zu hohe Zirkon-Konzentration bei der Herstellung der Magnesiumlegierung dazu führen würde, dass sich Mg-Fe-Partikel in der Schmelze bilden und aus der Schmelze ausfallen, was den Eisenanteil der Magnesiumlegierung ungewünscht verringern würde.

In einer weiteren Ausführungsform umfasst die Magnesiumlegierung ein oder mehrere Elemente der Gruppe Co, Ni mit einem jeweiligen Anteil von weniger als 100 ppm, bevorzugt weniger als 50ppm und/oder Be mit einem Anteil kleiner 20 ppm, bevorzugt kleiner 4ppm umfasst. Beispielsweise ist Nickel nicht biokompatibel. Beryllium ist extrem toxisch und sollte daher einen möglichst geringen Anteil in der Magnesiumlegierung besitzen. Berylliumspuren werden manchmal bei der Herstellung von reinem Magnesium beobachtet, da Beryllium die Oxidation von Magnesium in der Schmelze verhindert.

In einer Ausführungsform ist die Anordnung entweder federelastisch oder duktil ausgeführt. Eine elastische Anordnung, insbesondere das Bauteil, ermöglicht es, dass die Anordnung beziehungsweise das Bauteil entweder über eine Form gestülpt werden kann, damit es dann von außen fest an dieser Form anliegt oder dass die Anordnung zusammenzupresst werden kann, um die Anordnung in eine äußere Form einlegen zu können, damit die Anordnung dann nach elastischer Ausdehnung fest an der äußeren Form anliegt. Hierbei wird die Elastizität durch den Querschnitt, den Durchmesser der Anordnung beziehungsweise des Bauteil, die Temperatur bei der Verwendung und durch die Zusammensetzung des Materials bestimmt. Alternativ kann das Bauteil duktil ausgeführt sein, damit es formstabil in eine vorgesehene Position eingefügt werden kann. Formstabil bezeichnet dabei die Eigenschaft eines Bauteils ohne externe Krafteinwirkung, also nur unter Einwirkung Eigengewichts seine Form nicht oder nur minimal zu verändern. Die Duktilität bezeichnet dabei die Eigenschaft eines Werkstoffs, sich unter Belastung plastisch zu verformen, bevor der Werkstoff unter der Belastung versagt. Eine Magnesiumlegierung ist in einem Einkristall oder in einer nahezu einkristallinen Form federelastisch. Hierbei spielt die Textur des Bauteils aus der Magnesiumlegierung eine große Rolle bei der Größe der Elastizität. Die Textur kann durch die Materialherstellung an sich, als auch durch nachfolgende Umformprozesse wie die Extrusion- und Ziehprozesse bei der Herstellung des Bauteils kontrolliert werden. Mit einem sogenannten Bridgeman-Apparat lassen sich beispielsweise auch Magnesium-Einkristalle aufwachsen.

In einer weiteren Ausführungsform ist das Bauteil als flexibler Faden mit einem Durchmesser kleiner 0,6mm, bevorzugt zwischen 0,1mm und 0,4mm, ausgebildet. Ab einem Durchmesser von mehr als 0,6mm ist eine Anordnung zu dick und damit zu unhandlich für ihren Zweck als Implantat beziehungsweise als Zugabe zu einem Implantat, insbesondere wenn die Vermeidung von Periimplantitis bei dentalen Implantaten bezweckt ist. Beispielsweise besitzt die Anordnung einen Durchmesser von 0,3 mm. Der Durchmesser bezeichnet hier die laterale Ausdehnung des Bauteils beziehungsweise der Anordnung senkrecht zu ihrer Länge. In einer anderen Ausführungsform ist die Anordnung ein Nahtmaterial. Hier gilt das gleiche für den Durchmesser der Anordnung als Nahtmaterial. Fäden aus Magnesiumlegierungen als Anordnung beziehungsweise Bauteil lassen sich beispielsweise mittels Drahtziehen oder mittels Extrusion herstellen. Hierbei werden feste oder dickflüssige härtbare Materialien unter Druck kontinuierlich aus einer formgebenden Öffnung herausgezogen beziehungsweise herausgepresst. Dabei entstehen die resultierenden Körper mit dem Querschnitt der Öffnung in grundsätzlich beliebiger Länge.

In einer weiteren Ausführungsform ist das Bauteil als formstabile Form mit zumindest abgerundeten Kanten ausgebildet. Formstabile Körper können beispielsweise mittels Stanzen oder Laserschneiden mit einer sehr hohen Geschwindigkeit und je nach Prozessdesign in großen Stückzahlen parallel mit einer einzigen Stanzung oder einem einzigen Laser- oder Wasserstahlschneideprozess aus einer entsprechenden Platte aus der Magnesiumlegierung der Anordnung beziehungsweise des Bauteils hergestellt werden.

In einer weiteren Ausführungsform besitzt die formstabile Form eine kreisförmige Form mit einer Lücke und mit zwei der Lücke zugewandten Enden, wobei an den Enden jeweils ein Zug- oder Druckmittel angeordnet ist, um die Lücke mittels Ausübung einer Zug- oder Druckkraft an den Zug- oder Druckmitteln des Bauteils zumindest zeitweise zu vergrößern oder zu verkleinern. Dadurch wird ermöglicht, dass eine elastische Anordnung, insbesondere das Bauteil, trotz der formstabilen Form durch Ausübung einer Zugkraft geweitet und nachfolgend über eine andere Form, beispielsweise ein Zahn eines Patienten, gestülpt werden kann, damit die Anordnung dann ohne Zugkraft von außen fest an dieser Form, beispielsweise dem Zahn, anliegt. Dadurch wird auch ermöglicht, dass die elastische Anordnung, insbesondere das Bauteil, trotz der formstabilen Form durch Ausübung einer Druckkraft verkleinert und nachfolgend in die andere Form mit einem Hinterschnitt, beispielsweise die Zahntasche eines Patienten, eingebracht werden kann, damit die Anordnung dann ohne Druckkraft von innen fest in dieser Form, beispielsweise der Zahntasche, anliegt.

In einer Ausführungsform ist das Bauteil mit einer äußeren Beschichtung mit einer Dicke kleiner oder gleich 15µm, insbesondere kleiner oder gleich 10 µm, versehen. Die Beschichtung kann zusätzlich eine antibakterielle Wirkung aufweisen und/oder zur Herstellung einer glatten Oberfläche der Anordnung beziehungsweise des Bauteils dienen. Eine antibakterielle Beschichtung kann eine kontrollierte Freisetzung von biofunktionalen Substanzen bewirken, was den Heilungsprozess fördert. Die Beschichtung kann auch aus einem Material der Gruppe nativer oder künstlicher Kollagene, Fibrine, Fibroine, natürlicher oder künstlicher Seiden, natürlicher Proteine oder Polymere wie PLA, PDLA, PLLA, PTFE, eine Sol-Gel-Schicht hergestellt sein oder eine Oxidschicht, beispielweise hergestellt mittels plasmaelektrolytischer Oxidation, sein. Eine solche Beschichtung kann die Anordnung beziehungsweise das Bauteil schützen oder seine Oberflächeneigenschaften anpassen.

In einer weiteren Ausführungsform erfüllt die Anordnung die Funktion eines Hautexpanders. Gewebe- oder Hautexpander werden verwendet, um zum Beispiel bei Defekten durch Verbrennungen oder Unfälle Haut für die Transplantation zu gewinnen. Diese werden vorwiegend im Bereich Rekonstruktion, also in der Wiederherstellung eingesetzt. Ein Expander wird wie ein vorübergehendes Implantat eingesetzt. Üblicherweise ist ein Expander eine leere Hülle mit einem eingebauten Ventil, welche nach und nach mit Kochsalzlösung oder Luft aufgefüllt wird. Durch die langsame Dehnung der Haut über Wochen oder Monate wird zusätzliches Gewebe gewonnen, das an eine andere Stelle am Körper des Patienten transplantiert werden kann. Der Nachteil üblicher Expander ist, dass das Implantat nach der Hautdehnung wieder entfernt werden muss. Dies kann zwar im Rahmen der Hauttransplantation erfolgen, stellt aber einen weiteren Eingriff während dieser Operation dar. Besonders von Nachteil aber ist, dass während der Hautdehnung zur Befüllung der Hülle dauerhaft ein Katheter vorhanden ist, welcher durch den permanenten Hautdurchtritt ein erhöhtes Infektionsrisiko aufweist. Neuerdings wird das Infektionsrisiko zwar durch eine Hülle oder ein quellendes Kissen mit einer Injektionsmembran vermindert, da kein permanenter Zugang gewährleistet werden muss und die Befüllung durch das wiederkehrende Injizieren mit einer Nadel erfolgt. Nichtsdestotrotz stellt auch das regelmäßige Injizieren einer Nadel ein erhöhtes Infektionsrisiko dar, insbesondere für Patienten mit ohnehin erhöhtem Risiko, beispielweise Verbrennungsopfern, welche häufig einer Hauttransplantation bedürfen. Erfindungsgemäß löst die vorliegende Erfindung dieses Problem dadurch, dass die vorgeschlagene Anordnung in Form eines Bauteils aus einer schnell korrodierenden Magnesiumlegierungen an einer geeigneten Stelle unter die Haut implantiert wird und dort durch die exzessive Bildung von Wasserstoffgas über einen definierten Zeitraum hinweg ohne einen weiteren Eingriff eine Blase bildet, welche die Haut erweitert. Somit wirkt die Anordnung wie ein Expander, ohne die sonst üblichen Risiken aufzuweisen. Beispielsweise kann die Anordnung ein wirr abgewickelter flexibler Faden sein, welcher durch einen kleinen Schnitt unter die Haut des Patienten geschoben und diese dann wieder verschlossen wird, oder bei der Anordnung kann es sich um ein Bauteil mit einer formstabilen Form, beispielsweise ein spiralförmig aus einer Platte oder einer Folie mittels Stanzen oder Laserschneiden ausgeschnittenes Gebilde, handeln. Die Anordnung weist darüber hinaus zusätzlich die voranstehend beschriebene antibakterielle Wirkung auf, sodass das ohnehin verminderte Infektionsrisiko auf ein Minimum reduziert wird.

In einer weiteren Ausführungsform umfasst die Anordnung weitere flexible Fäden, wobei die weiteren Fäden und das Bauteil mit ihren Längsachsen parallel und symmetrisch zueinander als fadenförmige Anordnung angeordnet sind, wobei das Bauteil ebenfalls als flexibler Faden ausgeführt ist. Die resultierende Anordnung ist damit ebenfalls fadenförmig und flexibel. Die weiteren Fäden können dabei das Bauteil schützen oder eigene Funktionalitäten zusätzlich zu denen des Bauteils bereitstellen wie beispielsweise die bessere Verwendung bei bestimmten Frakturen, wo beispielsweise ein gerissenes Band wieder mit einem Knochen verbunden werden muss und diese Anordnung als besonders stabile Schnur oder Knoten mit zusätzlicher antibakterieller Wirkung verwendet wird oder wenn eine gewisse Beweglichkeit für das Einbringen das Bauteils erforderlich ist. In einer Ausführungsform sind dabei alle Fäden jeweils als erfindungsgemäße Bauteile ausgeführt und miteinander verdrillt, was die mechanische Stabilität der Anordnung erhöht.

In einer weitern Ausführungsform sind das Bauteil als mittlerer Faden und die weiteren Fäden als eine äußere erste Hülle in einer symmetrischen und parallelen Anordnung der weiteren Fäden zueinander um das Bauteil herum angeordnet. Hier können die Funktionalitäten der weiteren Fäden auf das Bauteil als mittleren Faden abgestimmt werden. Insbesondere kann die Geschwindigkeit der Resorption des Magnesiums aus der Magnesiumlegierung durch die weiteren Fäden nach Wunsch eingestellt werden. Beispielsweise können mehrere der weiteren Fäden als Bauteil aus der Magnesiumlegierung hergestellt sein, um die Abgabe von Silber an die umgebende Schleimhaut je nach Schweregrad der Entzündung zu verstärken, oder es können weitere Fäden mit einer geringeren Silberkonzentration oder gänzlich ohne Silber zur Verlangsamung der Silberabgabe um das Bauteil herum angeordnet sein. In einer weiteren Ausführungsform der fadenförmigen Anordnung sind noch weitere Fäden zumindest als eine weitere zweite Hülle umfasst, wobei diese weiteren Fäden mit Ihren Längsachsen symmetrische und parallele zueinander um die erste Hülle herum angeordnet sind. Dabei können zumindest einige der weiteren Fäden auch aus einem Fadenmaterial unterschiedlich zum Material des Bauteils ausgeführt, vorzugsweise ist das Fadenmaterial ein Material aus der Gruppe nativer oder künstlicher Kollagene, Fibrine, Fibroine, natürlicher oder künstlicher Seiden, natürlicher Proteine oder Polymere PLA, PDLA, PLLA, PTFE oder PCA oder das Fadenmaterial ist ein zumindest oberflächlich, vorzugsweise mittels plasmaelektrolytischer Oxidation, oxidiertes Material des Bauteils. Diese Oberflächenoxidschicht passiviert die Magnesiumlegierung des Bauteils gegenüber Umwelteinflüssen von außen auf das Bauteil. Die Oxidschicht kann dabei künstlich erzeugt oder natürlich gewachsen sein. Eine künstliche Oxidschicht kann beispielsweise mittels Anodisation oder plasmaelektrolytische Oxidation (PEO) erzeugt werden. Die plasmaelektrolytische Oxidation (PEO) wird unter anderem auch als plasmachemische Oxidation (PCO), Anodisation unter Funkenbildung (ANOF) oder als sogenannte Micro-Arc-Oxidation (MAO) bezeichnet. Die natürlich gewachsene Oxidschicht kann beispielsweise an Luft oder in einem Gas mit kontrolliertem Sauerstoffanteil aufwachsen. Die bevorzugte Dicke dieser Oxidschicht beträgt nicht mehr als 10µm. Bei Magnesiumlegierungen kann die Oxidschicht ein Auflösen des Bauteils in wässriger Umgebung verhindern oder den Auflösungsprozess zeitlich kontrollieren, was je nach Anwendung entscheidend sein kann, beispielsweise bei erfindungsgemäßen Anordnungen als Implantate. Die weiteren Fäden der zweiten Hülle können beispielsweise eine ästhetische Funktion bereitstellen, indem diese weiteren Fäden aus einem Polymer, beispielsweise PTFE, hergestellt sind und in Hautfarbe oder in der Farbe der jeweiligen Zähne bereitgestellt werden. Auf diese Weise ist die Anordnung am Lebewesen unauffällig nach außen. Die weiteren Fäden der ersten Hülle können dagegen beispielsweise die gewünschten angepassten antibakteriellen Eigenschaften der erfindungsgemäßen Anordnung bereitstellen. Hierbei können die weiteren Fänden in der ersten Hülle eine beliebige Farbe besitzen, da diese von der zweiten Hülle überdeckt werden und somit nach außen nicht sichtbar sind.

In einer weiteren Ausführungsform sind die weiteren Fäden der ersten und/oder zweiten Hülle miteinander um das Bauteil als den mittleren Faden herum verdrillt. Dies erhöht die mechanische Stabilität der Anordnung bei der Lagerung, dem Transport und bei der Verarbeitung in der jeweiligen Anwendung.

Die Erfindung betrifft des Weiteren eine Halterungsform mit einer durch die Halterungsform gehaltene erfindungsgemäße Anordnung umfassend mindestens ein Bauteil aus dem vorhergehend beschriebenen resorbierbaren Material mit antibakterieller Wirkung, wobei die Anordnung als flexible fadenförmige Anordnung mit einer Form mit einem Aspektverhältnis größer 100 ausgebildet ist und wobei die Halterungsform dazu vorgesehen ist, mittels Durchschneiden des Materials der Anordnung davon separate Anordnungen in einer gewünschten Länge mit kleinerem Aspektverhältnis, beispielsweise mit einem Aspektverhältnis von 10, zu erzeugen. Hier ist die Anordnung ebenfalls eine fadenförmige Anordnung, die entsprechend länger ist. Die Halterungsform kann eine Rolle sein, auf der die Anordnung aufgewickelt ist. Das Durchschneiden kann beispielsweise mit einer Schere oder einem Messer erfolgen.

### Kurze Beschreibung der Abbildungen

Diese und andere Aspekte der Erfindung werden im Detail in den Abbildungen wie folgt gezeigt:
- Fig.1:: eine Ausführungsform der erfindungsgemäßen Anordnung mit einem flexiblen fadenförmigen Bauteil;
- Fig.2:: eine Ausführungsform der erfindungsgemäßen Anordnung mit einem formstabilen kreisförmigen Bauteil;
- Fig.3:: eine weitere Ausführungsform der erfindungsgemäßen Anordnung umfassend das Bauteil als Faden und weitere Fäden, die alle miteinander verdrillt sind;
- Fig.4:: ein Schnitt durch eine Ausführungsform der erfindungsgemäßen Anordnung mit weiteren Fäden, die um das Bauteil als mittlerem Faden herum angeordnet sind;
- Fig.5:: eine Ausführungsform eines Fadens der erfindungsgemäßen Anordnung mit einem innerem Kern und einer Außenschicht;
- Fig.6:: eine Ausführungsform einer erfindungsgemäßen Halterungsform mit darauf gehaltener erfindungsgemäßer Anordnung;
- Fig.7:: die erfindungsgemäße Anordnung als Gewebe- oder Hautexpander oder als antibakterielles Implantat in Form eines Fadens in Draufsicht auf die Haut;
- Fig.8:: die erfindungsgemäße Anordnung als Hautexpander mit formstabiler Form im seitlichen Schnitt durch die Haut.

### Detaillierte Beschreibung der Ausführungsbeispiele

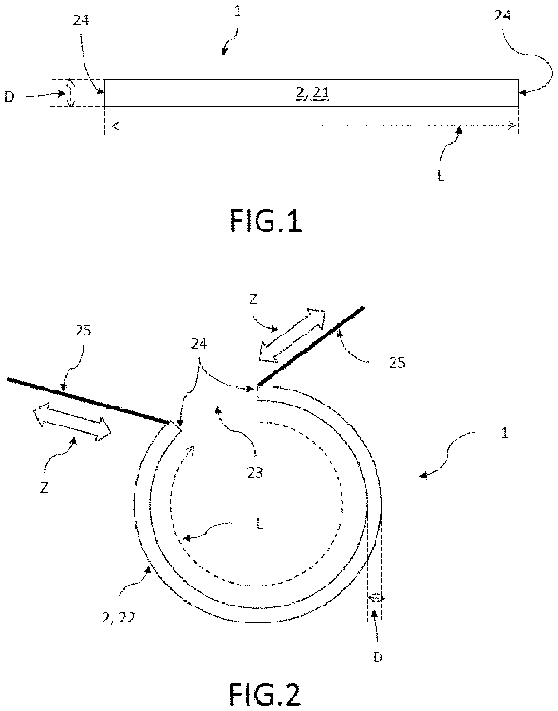

Fig.1 zeigt eine Ausführungsform der erfindungsgemäßen Anordnung 1 mit einem flexiblen fadenförmigen Bauteil 2, 21 aus einem resorbierbaren Material mit antibakterieller Wirkung. Das Bauteil hat hier die Form eines Fadens 21 mit einem Aspektverhältnis größer 10, wobei der Durchmesser zwischen 0,1 mm und 0,4mm beträgt. Das Aspektverhältnis ist dabei das Verhältnis zwischen Länge L des Fadens 21 und dem Durchmesser D des Fadens 21. Das Material des Bauteils 2, 21 ist eine schnell korrodierende Magnesiumlegierung mit einem Silberanteil zwischen 6% und 10% und mit einem Eisenanteil größer 100ppm und kleiner als 1%. In anderen Ausführungsformen könnte der Eisenanteil auch zwischen 250ppm und 0,5% liegen. Der Faden kann mit einer äußeren Beschichtung mit einer Dicke kleiner oder gleich 10µm versehen sein. In einer anderen Ausführungsform ist das Material des Bauteils 2, 21 eine schnell korrodierende Magnesiumlegierung beispielsweise mit einem Silberanteil zwischen 6% und 10% und mit einem Kupferanteil von mindestens 0,1%, besser größer 0,3% und kleiner als 10%. In anderen Ausführungsformen könnte der Kupferanteil auch zwischen 0,6% und 5% liegen.

Die Magnesiumlegierung des Bauteils 2, 21 kann als Zusatz ein oder mehrere Elemente der Gruppe Al, Zn, Si, Mn, Ca, Li, Sn, Sr und/oder P mit einem jeweiligen Anteil kleiner 1%, bevorzugt kleiner 0,6%, besonders bevorzugt kleiner 0,2% bei einem Gesamtanteil kleiner 2%, bevorzugt kleiner 1,5%, besonders bevorzugt kleiner 0,4% oder ein oder mehrere Elemente der Gruppe Sc, Y, La, Ce, Pr, Nd, Pm, Sm, Eu, Gd, Tb, Dy, Ho, Er, Tm, Yb, und/oder Lu mit einem jeweiligen Anteil kleiner 4,5%, bevorzugt kleiner 2%, besonders bevorzugt kleiner 0,15% mit einem Gesamtanteil kleiner 7,5%, bevorzugt kleiner 5%, besonders bevorzugt kleiner 0,4% umfassen. Die voranstehenden Angaben beziehen sich auf Gewichtsprozente. Die Magnesiumlegierung kann auch Zirkon mit einem Anteil kleiner 0,5% umfassen. Die Magnesiumlegierung kann auch ein oder mehrere Elemente der Gruppe Co, Ni mit einem jeweiligen Anteil von weniger als100ppm, bevorzugt weniger als 50ppm und/oder Be mit einem Anteil kleiner 20ppm, bevorzugt kleiner 4ppm umfassen.

Fig.2 zeigt eine Ausführungsform der erfindungsgemäßen Anordnung 1 mit einem formstabilen kreisförmigen Bauteil 2, 22 ebenfalls aus einem resorbierbaren Material mit antibakterieller Wirkung. Das Bauteil 2, 22 hat hier eine federelastische kreisförmige Form 22 mit abgerundeten Kanten mit einer Lücke 23 und mit zwei der Lücke 23 zugewandten Enden 24. An diesen Enden 24 ist jeweils ein Zug- bzw. Druckmittel 25 angeordnet, um die Lücke 23 mittels Ausübung einer Zug- beziehungsweise- Druckkraft Z über die Zugbeziehungsweise Druckmittel 25 des Bauteils 2 zumindest zeitweise zur Erleichterung der Anbringung beziehungsweise Positionierung der Anordnung zu vergrößern oder zu verkleinern. Das Aspektverhältnis ist dabei das Verhältnis zwischen Länge L der kreisförmigen Form 22 entlang ihrer Ausdehnung und dem Durchmesser D der kreisförmigen Form 22. Das Material des Bauteils 2, 22 ist eine schnell korrodierende Magnesiumlegierung mit einem Silberanteil zwischen 6% und 10% und mit einem Eisenanteil größer 100ppm und kleiner als 1%. In anderen Ausführungsformen könnte der Eisenanteil auch zwischen 150ppm und 0,5% oder zwischen 250ppm und 0,1% liegen. In einer weiteren Ausführungsform ist das Material des Bauteils 2, 22 eine schnell korrodierende Magnesiumlegierung mit einem Silberanteil zwischen 6% und 10% und mit einem Kupferanteil von mindestens 0,1%, besser größer 0,3% und kleiner als 10%. In anderen Ausführungsformen könnte der Kupferanteil auch zwischen 0,6% und 5% oder zwischen 1,0% und 2% liegen. Die kreisförmige Form 22 kann mit einer äußeren Beschichtung mit einer Dicke kleiner oder gleich 10µm versehen sein.

Die Magnesiumlegierung des Bauteils 2, 22 kann als Zusatz ein oder mehrere Elemente der Gruppe Al, Zn, Si, Mn, Ca, Li, Sn, Sr und/oder P mit einem jeweiligen Anteil kleiner 1%, bevorzugt kleiner 0,6%, besonders bevorzugt kleiner 0,2% bei einem Gesamtanteil kleiner 2%, bevorzugt kleiner 1,5%, besonders bevorzugt kleiner 0,4% oder ein oder mehrere Elemente der Gruppe Sc, Y, La, Ce, Pr, Nd, Pm, Sm, Eu, Gd, Tb, Dy, Ho, Er, Tm, Yb, und/oder Lu mit einem jeweiligen Anteil kleiner 4,5%, bevorzugt kleiner 2%, besonders bevorzugt kleiner 0,15% mit einem Gesamtanteil kleiner 7,5%, bevorzugt kleiner 5%, besonders bevorzugt kleiner 0,4% umfassen. Die voranstehenden Angaben beziehen sich auf Gewichtsprozente. Die Magnesiumlegierung kann auch Zirkon mit einem Anteil kleiner 0,5% umfassen. Die Magnesiumlegierung kann auch ein oder mehrere Elemente der Gruppe Cu, Co, Ni mit einem jeweiligen Anteil von weniger als100ppm, bevorzugt weniger als 50ppm und/oder Be mit einem Anteil kleiner 20ppm, bevorzugt kleiner 4ppm umfassen.

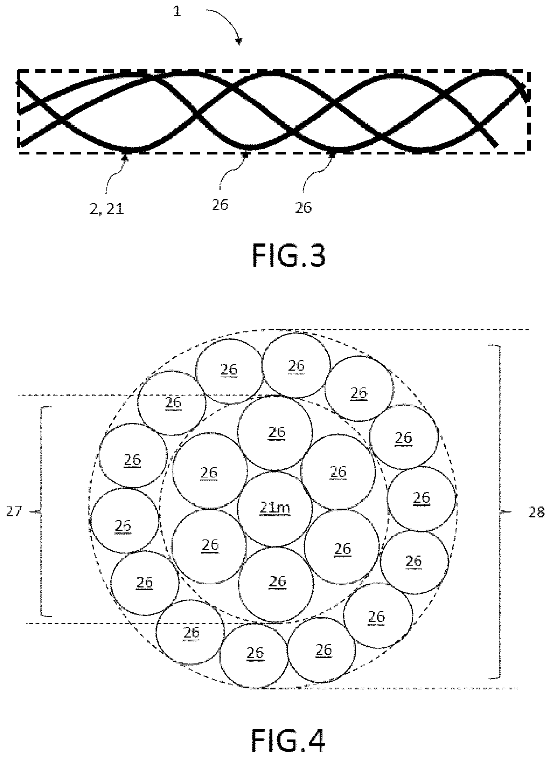

Fig.3 zeigt eine weitere Ausführungsform der erfindungsgemäßen Anordnung 1 umfassend das Bauteil 2 als flexiblen Faden 21 und weitere flexible Fäden 26, deren Längsachse parallel und symmetrisch zueinander als fadenförmige Anordnung 1 angeordnet sind, wobei das Bauteil 2, 21 und die Fäden 26 miteinander verdrillt sind.

Fig.4 zeigt einen Schnitt senkrecht zur Länge der Anordnung 1 durch eine Ausführungsform der erfindungsgemäßen Anordnung 1 mit weiteren Fäden 26, die um das Bauteil 2 als mittlerem Faden 21m als eine äußere erste Hülle 27 in einer symmetrischen und parallelen Anordnung zueinander um das Bauteil 21m herum angeordnet sind. Zusätzlich zur ersten Hülle 27 weist die fadenförmige Anordnung 1 hier noch weitere Fäden 26 als eine weitere zweite Hülle 28 mit einer symmetrischen und parallelen Anordnung der weiteren Fäden 26 zueinander um die erste Hülle 27 herum auf. Hierbei können zumindest einige der weiteren Fäden 26 oder gegebenenfalls auch alle weiteren Fäden aus einem Fadenmaterial unterschiedlich zum Material des Bauteils 21m ausgeführt sein. Das Fadenmaterial der weiteren Fäden 26 kann dabei ein Material aus der Gruppe nativer oder künstlicher Kollagene, Fibrine, Fibroine, natürlicher oder künstlicher Seiden, natürlicher Proteine oder Polymere PLA, PDLA, PLLA, PTFE oder PCA sein oder das Fadenmaterial ist ein zumindest oberflächlich, vorzugsweise mittels plasmaelektrolytischer Oxidation, oxidiertes Material des Bauteils 2. In einer hier nicht gezeigten Ausführungsform können die weiteren Fäden 26 der ersten und/oder zweiten Hülle 27, 28 miteinander um das Bauteil 2 als den mittleren Faden 21m herum verdrillt sein. In einer anderen Ausführungsform können die weiteren Fäden der ersten und zweiten Hülle auch aus einer Magnesiumlegierung bestehen oder diese zumindest umfassen. In einer zu Fig.4 alternativen Anordnung können die Fäden 21m, 26 auch umgekehrt angeordnet sein, sodass ein weiterer Faden 26 als zentraler Faden (analog zum mittleren Faden 21m in Fig.4) und das Bauteil 2 als Anordnung von Fäden (analog zu den weiteren Fäden in Fig.4) in einer äußeren ersten Hülle 27 in einer symmetrischen und parallelen Anordnung zueinander um den zentralen Faden herum angeordnet sind. Der zentrale Faden kann dabei die Festigkeit beziehungsweise Stabilität der Anordnung gewährleisten, während die Anordnung der Fäden des Bauteils 2 um den zentralen Faden herum ihre antibakterielle Wirkung entfaltet.

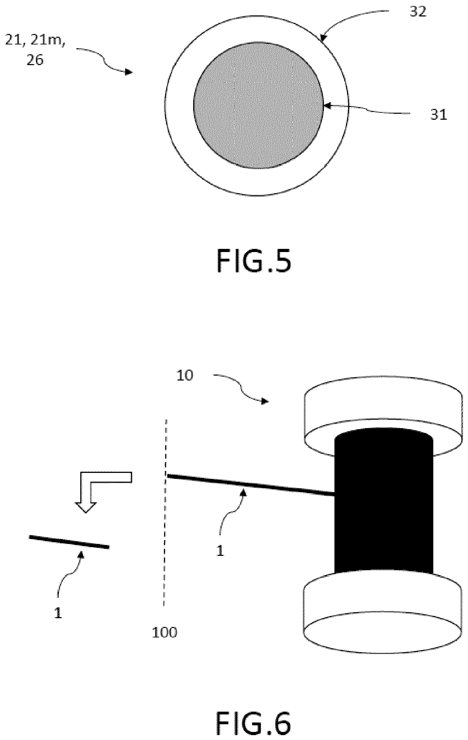

Fig.5 zeigt eine Ausführungsform eines Fadens 21, 21m, 26 der erfindungsgemäßen Anordnung mit einem inneren Kern 31 als tragende Struktur und einer Außenschicht 32. Der innere Kern 31 kann dabei aus der Magnesiumlegierung des Bauteils hergestellt sein oder im Falle als weiterer Faden 26 in manchen Ausführungsformen auch aus einem anderen Material bestehen. Die Außenschicht 32 kann aus einem anderen Material als der Kern 31 bestehen. Geeignete Materialien für die in Fig. 5 gezeigte Struktur (Kern 31, Außenschicht 32) sind unter anderen beispielsweise in Verbindung mit den Fig.1 und 4 beschrieben. Beispielsweise kann der innere Kern 31 auch der erfindungsgemäßen Magnesiumlegierung mit einer ersten Zusammensetzung und die Außenschicht 32 ebenfalls aus der erfindungsgemäßen Magnesiumlegierung mit einer zweiten Zusammensetzung ungleich der ersten Zusammensetzung bestehen.

Fig.6 zeigt eine Ausführungsform einer erfindungsgemäßen Halterungsform 10 mit darauf gehaltener erfindungsgemäßer Anordnung 1, die mindestens ein Bauteil 2 aus einem resorbierbaren Material mit antibakterieller Wirkung umfasst, wobei die Anordnung 1 als flexible fadenförmige Anordnung mit einer Form mit einem Aspektverhältnis größer einhundert ausgebildet ist und wobei die Halterungsform 10 dazu vorgesehen ist, mittels Durchschneiden 100 des Materials der Anordnung 1 davon separate Anordnungen 1 in einer gewünschten Länge mit kleinerem Aspektverhältnis zu erzeugen. Das mit der gestrichelten Linie angedeutete Durchschneiden 100 kann beispielsweise mit einer Schere oder einem Messer durchgeführt werden.

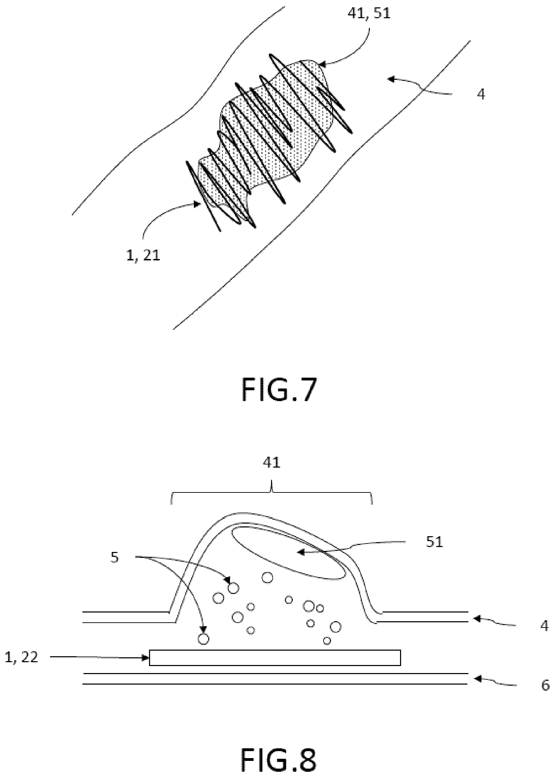

Fig.7 zeigt die erfindungsgemäße Anordnung 1 als Gewebe- oder Hautexpander in Form eines Fadens 21 in Draufsicht auf die Haut 4. Hier wurde die Anordnung 1 als wirr abgewickelter flexibler Faden 21 durch einen kleinen Schnitt unter die Haut 4 des Patienten geschoben (implantiert) und diese dann wieder verschlossen. Die schnell korrodierende Magnesiumlegierung der Anordnung 1 soll dort durch die exzessive Bildung von Wasserstoffgas (hier nicht gezeigt, siehe dazu Fig.8) über einen definierten Zeitraum die Haut 4 in dem zu dehnenden Bereich 41 als Blase 51 erweitern.

Fig.7 zeigt alternativ die erfindungsgemäße Anordnung 1 als antibakterielles Implantat in Form eines Fadens 21 in Durchsicht auf die Haut bzw. Draufsicht auf den Knochen 4. Hier wurde die Anordnung 1 als wirr abgewickelter flexibler Faden 21 durch einen kleinen Schnitt unter die Haut des Patienten geschoben (implantiert) und diese dann wieder verschlossen. Die schnell korrodierende Magnesiumlegierung der Anordnung 1 soll dort durch die Bildung von Wasserstoffgas und optional die Freisetzung von Silber-Ionen über einen definierten Zeitraum auf den infizierten Bereich 41 des Knochens 4 antibakteriell einwirken.

Fig.8 zeigt die erfindungsgemäße Anordnung 1 als Hautexpander mit formstabiler Form 22 im seitlichen Schnitt durch die Haut 4. Die formstabile Form 22 ist beispielsweise ein spiralförmig aus einer Platte oder einer Folie mittels Stanzen oder Laserschneiden ausgeschnittenes Gebilde, das durch einen Schnitt unter die Haut 4 des Patienten geschoben (implantiert) und diese dann wieder verschlossen wird. Wie auch in der Ausführungsform gemäß Fig.7 bildet die schnell korrodierende Magnesiumlegierung exzessiv Wasserstoffgas 5, das aufsteigt und über einen definierten Zeitraum hinweg ohne einen weiteren Eingriff eine Blase 51 aus Wasserstoffgas 5 bildet, welche die Haut 4 erweitert. Die Schicht 6 unterhalb der Anordnung 1 stellt hier subkutanes Gewebe dar.

Die Anordnung gemäß Fig. 7 und 8 weisen aufgrund der exzessiven Wasserstoffbildung die antibakterielle Wirkung auf, sodass das ohnehin verminderte Infektionsrisiko bei dieser Ausführungsform des Hautexpanders auf ein Minimum reduziert wird.

Die hier gezeigten Ausführungsbeispiele stellen nur Beispiele für die vorliegende Erfindung dar und dürfen daher nicht einschränkend verstanden werden.

### Liste der Bezugszeichen

- 1: erfindungsgemäße Anordnung
- 2: Bauteil der erfindungsgemäßen Anordnung
- 21: Bauteil als flexibler Faden
- 21m: Bauteil als flexibler mittlerer Faden
- 22: Bauteil als formstabile Form
- 23: Lücke im formstabilen Bauteil
- 24: Enden des Bauteils
- 25: Zugmittel am formstabilen Bauteil
- 26: weitere Fäden in der erfindungsgemäßen Anordnung
- 27: erste Hülle der weiteren Fäden in der erfindungsgemäßen Anordnung
- 28: zweite Hülle der weiteren Fäden in der erfindungsgemäßen Anordnung
- 31: innerer Kern eines Fadens in der erfindungsgemäßen Anordnung
- 32: Außenschicht eines Fadens in der erfindungsgemäßen Anordnung
- 4: Haut oder Knochen
- 41: zu dehnender bzw. gedehnter Bereich der Haut bzw. infizierter Bereich des Knochens
- 5: Wasserstoffgas
- 51: Blase aus Wasserstoffgas
- 6: subkutanes Gewebe
- 10: Halterungsform, beispielsweise eine Rolle
- 100: Durchschneiden der auf der Halterungsform gehaltenen Anordnung

- D: Durchmesser des Bauteils
- L: Länge des Bauteils
- Z: Zugkraft

## Patentansprüche

1. Eine Anordnung (1) zur Verwendung als Gewebeexpander, Hautexpander oder Implantat mit antibakterieller Wirkung umfassend mindestens ein Bauteil (2) aus einem resorbierbaren Material mit antibakterieller Wirkung mit einer Form, die ein Aspektverhältnis größer 10 besitzt und wobei das Material eine schnell korrodierende Magnesiumlegierung ist, die
a. einen Silberanteil von zwischen 6% und 12%, und
b. einen Eisenanteil von zwischen 0,01 und 1% oder einen Kupferanteil von zwischen 0,1 und 10% umfasst.

2. Die Anordnung (1) nach Anspruch 1,
**dadurch gekennzeichnet, dass**
die Magnesiumlegierung einen Silberanteil zwischen 6% und 10%, umfasst.

3. Die Anordnung (1) nach Anspruch 1 oder 2,
**dadurch gekennzeichnet, dass**
die Magnesiumlegierung einen Eisenanteil kleiner als 1% und größer als 150 ppm, bevorzugt größer 200 ppm, und bevorzugt größer 250 ppm, umfasst.

4. Die Anordnung (1) nach Anspruch 1 bis 3,
**dadurch gekennzeichnet, dass**
der Eisenanteil zwischen 0,025% und 0,5% ist.

5. Die Anordnung (1) nach einem der voranstehenden Ansprüche,
**dadurch gekennzeichnet, dass**
die Magnesiumlegierung einen Kupferanteil kleiner als 10% und größer als 0,3%, bevorzugt größer 0,6%, und besonders bevorzugt größer 1,0%, umfasst.

6. Die Anordnung (1) nach einem der voranstehenden Ansprüche,
**dadurch gekennzeichnet, dass**
der Kupferanteil größer als 0,1% und kleiner 5%, besonders bevorzugt kleiner 2%, ist.

7. Die Anordnung (1) nach einem der voranstehenden Ansprüche,
**dadurch gekennzeichnet, dass**
die Magnesiumlegierung als Zusatz ein oder mehrere Elemente der Gruppe Al, Zn, Si, Mn, Ca, Li, Sn, Sr und/oder P mit einem jeweiligen Anteil kleiner 1%, bevorzugt kleiner 0,6%, besonders bevorzugt kleiner 0,2% und einem Gesamtanteil kleiner 2%, bevorzugt kleiner 1,5%, besonders bevorzugt kleiner 0,4% oder ein oder mehrere Elemente der Gruppe Sc, Y, La, Ce, Pr, Nd, Pm, Sm, Eu, Gd, Tb, Dy, Ho, Er, Tm, Yb, und/oder Lu mit einem jeweiligen Anteil kleiner 4,5%, bevorzugt kleiner 2%, besonders bevorzugt kleiner 0,15% umfasst, wobei der Gesamtanteil kleiner 7,5%, bevorzugt kleiner 5%, besonders bevorzugt kleiner 0,4% ist.

8. Die Anordnung (1) nach einem der voranstehenden Ansprüche,
**dadurch gekennzeichnet, dass**
die Magnesiumlegierung ein oder mehrere Elemente der Gruppe Co, Ni mit einem jeweiligen Anteil von weniger 100 ppm, bevorzugt weniger als 50ppm und/oder Be mit einem Anteil kleiner 20 ppm, bevorzugt kleiner 4 ppm umfasst.

9. Die Anordnung (1) nach einem der voranstehenden Ansprüche,
**dadurch gekennzeichnet, dass**
das Bauteil (2) als flexibler Faden (21) mit einem Durchmesser (D) kleiner 0,6 mm, bevorzugt zwischen 0,1 mm und 0,4 mm, ausgebildet ist, vorzugsweise ist die Anordnung (1) ein Nahtmaterial.

10. Die Anordnung (1) nach einem der Ansprüche 1 bis 8,
**dadurch gekennzeichnet, dass**
das Bauteil (2) als formstabile Form (22) mit zumindest abgerundeten Kanten ausgebildet ist und die formstabile Form (22) eine kreisförmige Form mit einer Lücke (23) und mit zwei der Lücke (23) zugewandten Enden (24) besitzt, wobei an den Enden (24) jeweils ein Zug- oder Druckmittel (25) angeordnet ist, um die Lücke (23) mittels Ausübung einer Zug- oder Druckkraft (Z) an den Zug- oder Druckmitteln (25) des Bauteils (2) zumindest zeitweise zu vergrößern oder zu verkleinern.

11. Die Anordnung (1) nach einem der voranstehenden Ansprüche,
**dadurch gekennzeichnet, dass**
das Bauteil mit einer äußeren Beschichtung mit einer Dicke kleiner oder gleich 15 µm, insbesondere kleiner oder gleich 10 µm versehen ist, vorzugsweise ist die Beschichtung aus einem Material aus der Gruppe nativer oder künstlicher Kollagene, Fibrine, Fibroine, natürlicher oder künstlicher Seiden, natürlicher Proteine oder Polymere PLA, PDLA, PLLA, PTFE, PCA, eine Sol-Gel-Schicht oder eine Oxidschicht, vorzugsweise hergestellt mittels plasmaelektrolytischer Oxidation.

12. Die Anordnung (1) nach Anspruch 10,
**dadurch gekennzeichnet, dass**
die Anordnung (1) ein Gewebe- oder Hautexpander ist.

13. Die Anordnung (1) nach einem der Ansprüche 1 bis 9,
**dadurch gekennzeichnet, dass**
die Anordnung (1) weitere flexible Fäden (26) umfasst, wobei die weiteren Fäden (26) und das Bauteil (2) mit ihren Langsachsen parallel und symmetrisch zueinander als fadenförmige Anordnung (1) angeordnet sind, wobei das Bauteil (2) ebenfalls als flexibler Faden (21) ausgeführt ist.

14. Die Anordnung (1) nach Anspruch 13,
**dadurch gekennzeichnet, dass**
alle Fäden (21, 26) jeweils als Bauteile (2) ausgeführt und miteinander verdrillt sind; oder dass das Bauteil (2) als mittlerer Faden (21m) und die weiteren Fäden (26) als eine äußere erste Hülle (27) in einer symmetrischen und parallelen Anordnung der weiteren Fäden (26) zueinander um das Bauteil (2, 21m) herum angeordnet sind, vorzugsweise umfasst dabei die parallele Anordnung noch weitere Fäden (26) zumindest als eine weitere zweite Hülle (28) umfasst, wobei diese weiteren Fäden (26) mit ihren Längsachsen symmetrisch und parallel zueinander um die erste Hülle (27) herum angeordnet sind.

15. Die Anordnung (1) nach Anspruch 14,
**dadurch gekennzeichnet, dass**
zumindest einige der weiteren Fäden (26) aus einem Fadenmaterial unterschiedlich zum Material des Bauteils (2, 21m) ausgeführt sind, vorzugsweise ist das Fadenmaterial ein Material aus der Gruppe nativer oder künstlicher Kollagene, Fibrine, Fibroine, natürlicher oder künstlicher Seiden, natürlicher Proteine oder Polymere PLA, PDLA, PLLA, PTFE oder PCA oder das Fadenmaterial ist ein zumindest oberflächlich, vorzugsweise mittels plasmaelektrolytischer Oxidation, oxidiertes Material des Bauteils (2).

16. Die Anordnung (1) nach Anspruch 14 oder 15,
**dadurch gekennzeichnet, dass**
die weiteren Fäden (26) der ersten und/oder zweiten Hülle (27, 28) miteinander um das Bauteil (2) als den mittleren Faden (21m) herum verdrillt sind.

17. Eine Halterungsform (10) mit einer durch die Halterungsform (10) gehaltene Anordnung (1) umfassend mindestens ein Bauteil (2) aus einem resorbierbaren Material mit antibakterieller Wirkung nach Anspruch 1, wobei die Anordnung (1) als flexible fadenförmige Anordnung mit einer Form mit einem Aspektverhältnis größer 100 ausgebildet ist und wobei die Halterungsform (10) dazu vorgesehen ist, mittels Durchschneiden (100) des Materials der Anordnung (1) davon separate Anordnungen (1) in einer gewünschten Lange mit kleinerem Aspektverhältnis zu erzeugen.

## Claims

1. An arrangement (1) for use as a tissue expander, skin expander or implant with antibacterial activity comprising at least one member (2) from a bioabsorbable material with antibacterial activity having a shape with an aspect ratio greater than 10 and wherein the material is a rapidly corroding magnesium alloy having
a. a silver content of between 6% and 12%; and
b. an iron content of between 0.01 and 1% or a copper content of between 0.1 and 10%.

2. The arrangement (1) according to claim 1,
**characterized in that**
the magnesium alloy has a silver content of between 6% and 10%.

3. The arrangement (1) according to claim 1 or 2,
**characterized in that**
the magnesium alloy has an iron content of less than 1% and greater than 150 ppm, preferably greater than 200 ppm, and preferably greater than 250 ppm.

4. The arrangement (1) according to claims 1 to 3,
**characterized in that**
the iron content is between 0.025% and 0.5%.

5. The arrangement (1) according to any one of the preceding claims,
**characterized in that**
the magnesium alloy has a copper content of less than 10% and greater than 0.3%, preferably greater than 0.6%, and particularly preferred greater than 1.0%.

6. The arrangement (1) according to any one of the preceding claims,
**characterized in that**
the copper content is greater than 0.1% and less than 5%, particularly preferred less than 2%.

7. The arrangement (1) according to one of the preceding claims,
**characterized in that**
the magnesium alloy contains one or more elements of the group Al, Zn, Si, Mn, Ca, Li, Sn, Sr and/or P with a respective content of less than 1%, preferably less than 0.6%, particularly preferred less than 0.2% and a total amount of less than 2%, preferably less than 1.5%, particularly preferred less than 0.4% or one or more Elements of the group Sc, Y, La, Ce, Pr, Nd, Pm, Sm, Eu, Gd, Tb, Dy, Ho, Er, Tm, Yb and/or Lu with a respective content of less than 4.5%, preferably less than 2%, particularly preferred less than 0.15%, whereby the total amount is less than 7.5%, preferably less than 5%, particularly preferred less than 0.4%.

8. The arrangement (1) according to any one of the preceding claims,
**characterized in that**
the magnesium alloy contains one or more elements of the group Co, Ni with a respective content of less than 100 ppm, preferably less than 50 ppm, and/or Be with a content of less than 20 ppm, preferably less than 4 ppm.

9. The arrangement (1) according to one of the preceding claims,
**characterized in that**
at least one member (2) is formed as a flexible thread (21) with a diameter (D) smaller than 0.6 mm, preferably between 0.1 mm and 0.4 mm, preferably the arrangement (1) is a suture material.

10. The arrangement (1) according to one of claims 1 to 8,
**characterized in that**
the member (2) is formed as a dimensionally stable form (22) with at least rounded edges and that the dimensionally stable form (22) has a circular form with a gap (23) and two ends (24) facing the gap (23), whereby traction or pressure fixtures (25) are each arranged at the ends (24), in order to increase or decrease at least temporarily the gap (23) by tensile or compressive force (Z) on the fixtures (25) of the member (2).

11. The arrangement (1) according to any one of the preceding claims,
**characterized in that**
the member (2) is provided with an outer coating with a thickness less than or equal to 15 µm, particularly less than or equal to 10 µm, preferentially from a material from the group of native or artificial collagens, fibrins, fibroins, natural or artificial silks, natural proteins or polymers PLA, PDLA, PLLA, PTFE, PCA, sol-gel layers or an oxide layer, preferably produced by plasma electrolytic oxidation.

12. The arrangement (1) according to claim 10,
**characterized in that**
the arrangement (1) is a tissue or skin expander.

13. The arrangement (1) is according to any one of claims 1 to 9,
**characterized in that**
the arrangement (1) comprises further flexible threads (26), wherein the further threads (26) and the member (2) are arranged with their longitudinal axes in parallel and symmetrical to each other as a thread-like arrangement (1), whereby the member (2) is also designed as a flexible thread (21).

14. The arrangement (1) according to claim 13,
**characterized in that**
all threads (21, 26) are each designed as members (2) and twisted together or **in that** the member (2) is designed as a middle thread (21m) and the further threads (26) form an outer first sheath (27) in a parallel and symmetrical arrangement of the further threads (26) to each other, positioned around the member (2, 21m), whereby preferably the parallel arrangement includes additional threads (26) in at least one further second sheath (28), whereby the said further threads (26) are parallelly and symmetrically arranged around the first outer sheath (27).

15. The arrangement (1) according to claim 14,
**characterized in that**
at least some of the further threads (26) are from a material different to the material of the member (2, 21m), preferably from a material of the group of native or artificial collagens, fibrins, fibroins, natural or artificial silks, natural proteins or polymers PLA, PDLA, PLLA, PTFE, PCA or being a part of the member (2) having a oxidized surface, preferably by means of plasma electrolytic oxidation.

16. The arrangement (1) according to claim 14 or 15,
**characterized in that**
the further threads (26) of the first and/or second sheath (27, 28) are twisted together around the member (2) being the middle thread (21m).

17. A fixture (10) holding an arrangement (1) comprising at least one member (2) of a resorbable material with antibacterial activity according to claim 1, wherein the arrangement (1) is designed as a flexible thread-like arrangement having a shape with an aspect ratio greater 100 and wherein the fixture (10) is intended to allow for the separation of different assemblies (1) with smaller aspect ratio in a desired length by cutting through the material (100) of the arrangement (1).

## Revendications

1. Dispositif (1) destiné à être utilisé comme prothèse d'expansion tissulaire, prothèse d'expansion cutanée ou implant avec activité anti-bactérienne, comprenant au minimum un composant (2) constitué d'un matériau résorbable avec activité anti-bactérienne, d'une forme ayant un rapport d'aspect supérieur à 10 et dont le matériau est un alliage de magnésium rapidement corrosif qui comporte
a. une teneur en argent comprise entre 6 et 12 %, et
b. une teneur en fer comprise entre 0,01 et 1 % ou une teneur en cuivre comprise entre 0,1 et 10 %.

2. Le dispositif (1) selon la revendication 1,
**caractérisé en ce que**
l'alliage de magnésium comporte une teneur en argent entre 6 et 10 %.

3. Le dispositif (1) selon la revendication 1 ou 2,
**caractérisé en ce que**
l'alliage de magnésium comporte une teneur en fer inférieure à 1 % et supérieure à 150 ppm, de préférence supérieure à 200 ppm et de préférence supérieure à 250 ppm.

4. Le dispositif (1) selon la revendication 1 à 3,
**caractérisé en ce que**
la teneur en fer est entre 0,025 et 0,5 %.

5. Le dispositif (1) selon l'une quelconque des revendications précédentes,
**caractérisé en ce que**
l'alliage de magnésium comporte une teneur en cuivre inférieure à 10 % et supérieure à 0,3 %, de préférence supérieure à 0,6 % et plus préférablement supérieure à 1,0 %.

6. Le dispositif (1) selon l'une quelconque des revendications précédentes,
**caractérisé en ce que**
la teneur en cuivre est supérieure à 0,1 % et inférieure à 5 % et plus préférablement inférieure à 2 %.

7. Le dispositif (1) selon l'une quelconque des revendications précédentes,
**caractérisé en ce que**
l'alliage de magnésium contient un ou plusieurs éléments du groupe Al, Zn, Si, Mn, Ca, Li, Sn, Sr et/ou P dans une proportion respective inférieure à 1 %, de préférence inférieure à 0,6 % et plus préférablement inférieure à 0,2 % et une teneur totale inférieure à 2 %, de préférence inférieure à 1,5 % et plus préférablement inférieure à 0,4 % ou un ou plusieurs éléments du groupe Sc, Y, La, Ce, Pr, Nd, Pm, Sm, Eu, Gd, Tb, Dy, Ho, Er, Tm, Yb et/ou Lu dans une proportion respective inférieure à 4,5 %, de préférence inférieure à 2 % et plus préférablement inférieure à 0,15 %, la teneur totale étant inférieure à 7,5 %, de préférence inférieure à 5 % et plus préférablement inférieure à 0,4 %.

8. Le dispositif (1) selon l'une quelconque des revendications précédentes,
**caractérisé en ce que**
l'alliage de magnésium comporte un ou plusieurs éléments du groupe Co, Ni dans une proportion respective inférieure à 100 ppm, de préférence inférieure à 50 ppm et/ou Be dans une proportion inférieure à 20 ppm, de préférence inférieure à 4 ppm.

9. Le dispositif (1) selon l'une quelconque des revendications précédentes,
**caractérisé en ce que**
le composant (2) est conçu comme un fil flexible (21) d'un diamètre (D) inférieur à 0,6 mm, de préférence entre 0,1 mm et 0,4 mm, le dispositif (1) étant de préférence un matériel de suture.

10. Le dispositif (1) selon la revendication 1 à 8,
**caractérisé en ce que**
le composant (2) est conçu comme une pièce (22) de forme stable avec au minimum des bords arrondis et **en ce que** la pièce (22) de forme stable est circulaire avec un interstice (23) et deux extrémités (24) tournées vers l'interstice (23), un moyen de traction ou de compression (25) étant placé aux extrémités (24) pour agrandir ou diminuer, pour le moins temporairement, l'interstice (23) sous l'effet d'une force de traction ou de compression (Z) sur le moyen de traction ou de compression (25) du composant (2).

11. Le dispositif (1) selon l'une quelconque des revendications précédentes,
**caractérisé en ce que**
le composant est pourvu d'un revêtement extérieur d'une épaisseur inférieure ou égale à 15 µm, notamment inférieure ou égale à 10 µm, de préférence constitué d'un matériau appartenant au groupe des collagènes natifs ou artificiels, des fibrines, des fibroïnes, des soies naturelles ou artificielles, des protéines naturelles ou des polymères PLA, PDLA, PLLA, PTFE, PCA ou une couche sol-gel ou une couche d'oxyde, ayant été de préférence obtenue par oxydation par plasma électrolytique.

12. Le dispositif (1) selon la revendication 10,
**caractérisé en ce que**
le dispositif (1) est une prothèse d'expansion tissulaire ou cutanée.

13. Le dispositif (1) selon la revendication 1 à 9,
**caractérisé en ce que**
le dispositif (1) comprend des autres fils souples (26), les axes longitudinaux des autres fils (26) et du composant (2) étant orientés parallèlement et symétriquement entre eux pour former un dispositif (1) filiforme, le composant (2) étant également un fil souple (21).

14. Le dispositif (1) selon la revendication 13,
**caractérisé en ce que**
tous les fils (21, 26) sont conçus comme des composants (2) et sont tous torsadés ensemble ou **en ce que** le composant (2) est conçu en tant que fil central (21 m) et les autres fils (26) comme première gaine extérieure (27), les autres fils (26) étant disposés autour du composant (2, 21m) symétriquement et parallèlement entre eux, de préférence le dispositif parallèle inclut d'autres fils (26) dans au moins une deuxième gaine (28), les axes longitudinaux de ces autres fils (26) étant orientés symétriquement et parallèlement entre eux autour de la première gaine (27).

15. Le dispositif (1) selon la revendication 14,
**caractérisé en ce que**
au minimum certains autres fils (26) sont fabriqués dans un matériau de fil différent du matériau du composant (2, 21 m), le matériau de fil de préférence constitué d'un matériau appartenant au groupe des collagènes natifs ou artificiels, des fibrines, des fibroïnes, des soies naturelles ou artificielles, des protéines naturelles ou des polymères tels que PLA, PDLA, PLLA, PTFE, PCA ou **en ce que** le fil est au minimum dans le matériau du composant (2), oxydé en surface, de préférence par oxydation par plasma électrolytique.

16. Le dispositif (1) selon la revendication 14 ou 15,
**caractérisé en ce que**
les autres fils (26) de la première et/ou de la seconde gaine (27, 28) sont torsadés les uns avec les autres autour du composant (2) formant le fil central (21 m).

17. Une pièce de maintien (10) retenant un dispositif (1) comportant au minimum un composant (2) constitué d'un matériau résorbable avec activité anti-bactérienne selon la revendication 1, dans lequel le dispositif (1) est un dispositif filiforme souple d'une forme ayant un rapport d'aspect supérieur à 100, la pièce de maintien (10) étant prévue pour permettre la séparation de différents dispositifs (1) dans une longueur souhaitée avec un rapport d'aspect inférieur, par découpe (100) du matériau du dispositif (1).
